# EUROPEAN PATENT APPLICATION

(11) **EP 3 248 589 A1**
(43) Date of publication of application: **29.11.2017**
(21) Application number: 16740247.8
(22) Date of filing: 21.01.2016
(51) Int. Cl.: A61K 8/49, A61K 8/19, A61K 8/24, A61K 8/25, A61K 8/27, A61K 8/29, A61K 8/92, A61Q 1/04, A61Q 1/08, A61Q 1/10

(54) **MAKEUP COSMETIC**

(30) Priority: 21.01.2015 JP 2015009872; 19.01.2016 JP 2016007821
(71) Applicant: Shiseido Company Ltd., Chuo-ku Tokyo 104-0061 (JP)
(72) Inventor: OOHASHI, Shihoka, Yokohama-shi Kanagawa 224-8558 (JP); HIRUMA, Yukiko, Yokohama-shi Kanagawa 224-8558 (JP)
(74) Representative: Merkle, Gebhard
(86) International application number: PCT/JP2016/051682
(87) International publication number: WO 2016/117640

(57) **Abstract**

An object of the present invention is to provide a novel makeup cosmetic successfully producing a bright fluorescent color and providing transparency (clearness). The present invention provides a makeup cosmetic comprising: (A) at least one or two or more kinds of oil-soluble dye selected from the group consisting of Red No. 218, Red No. 223 and Orange No. 201, (B) a particle of a specific material selected from hydroxyapatite, calcium carbonate, a metal oxide (except iron oxide) and silica, and (C) an oily component, wherein the content of a pigment is 0.5 mass% or less; and the content of water is less than 1.5 mass%. The makeup cosmetic of the present invention can produce a bright fluorescent color as well as transparency, and is suitable as a lipstick, a blusher or an eyeshadow.

## Description

### Technical Field

The present invention relates to a makeup cosmetic capable of emitting fluorescence to provide a bright (brilliant) fluorescent color and transparent (clear) finish. More specifically, the present invention relates to a cosmetic coloring material containing a predetermined material(s), an oil-soluble dye and an oily component in combination and a makeup cosmetic containing the cosmetic coloring material.

### Background Art

Makeup cosmetics including lip cosmetics for coloring lips such as lipsticks and glosses, blushers (rouges) and eye shadows exert important cosmetic effects in significantly changing the physical appearance of the user. The color of a cosmetic is a key factor in choosing a cosmetic product. Recently, it has been desired to develop makeup cosmetics having a bright color and giving a transparent (clear) impression; for example, a lipstick displaying a highly bright fluorescent color has attracted attention.

Makeup cosmetics usually contain an oil base and a coloring material (coloring agent). The coloring material is responsible for producing a color when the cosmetic is applied to the lips and skin. The coloring materials to be blended in cosmetics include organic synthetic coloring agents (also called as tar colors) and inorganic pigments. The organic synthetic coloring agents are classified into dyes and organic pigments. The organic pigments are further classified into pigment coloring agents themselves and lake pigments, which are prepared by making water-soluble or sparingly-water-soluble dyes to be water insoluble (Non Patent Document 1).

Patent Document 1 discloses that if a fluorescent lame (lamé), which is prepared by adding flaky powder having at least one resin layer stained with a fluorescent dye or a fluorescent pigment, is blended in a makeup cosmetic, the fluorescent color of the resultant cosmetic is improved in uniformity and stability compared to the case in which a fluorescent dye or a fluorescent pigment is directly blended in a cosmetic.

However, it is considered that the color in appearance is determined by a pigment (Non Patent Document 1). The pigment, which is usually blended at 1 mass% or more, determines the color in appearance and also produces covering power. The cosmetic disclosed in Patent Document 1, which is prepared by adding a fluorescent lame containing a dye or pigment producing a fluorescent color to the color in appearance previously determined by a pigment, is interpreted as a makeup cosmetic for changing appearance. A dye or pigment intrinsically producing a fluorescent color is used as a material for the lame.

Patent Document 2 focuses on an oil-soluble dye for producing color of a lipstick cosmetic; however, the literature only discloses a technique for blending perfluoropolyether in combination with an oil-soluble dye in order to solve problems, i.e., chapped lips and color change in appearance caused by dyeing of an oil-soluble dye conventionally used, and is silent for a fluorescent color produced by the oil-soluble dye.

Patent Document 3 discloses that a makeup cosmetic (including a lipstick) containing an oil-soluble dye, which does not produce a fluorescent color when it is dissolved or dispersed alone in an oily component, in combination with a polyamide powder having a predetermined molecular weight and a predetermined particle diameter, produces a fluorescent color. However, the cosmetic containing an oil-soluble dye and a polyamide powder has a high covering power and lacks in transparency.

In short, a makeup cosmetic successfully producing a bright fluorescent color and providing transparency (clearness) has not yet been obtained up to present.

### Relevant Art Documents

### Patent Documents

Patent Document 1: JP-A 2004-346025
Patent Document 2: JP-B 3409191
Patent Document 3: JP-B 5312738

### Non Patent Document

Non Patent Document 1: "New Cosmetology (second edition)", edited by Takeo Mitsui, Nanzando Co., Ltd., 2001

### Summary of Invention

### Technical Problem

An object of the present invention is to provide a novel makeup cosmetic successfully producing a bright fluorescent color and providing transparency (clearness).

### Solution to Problem

The present inventors conducted intensive studies with a view to solving the aforementioned problems and, as a result, found that a makeup cosmetic producing a bright fluorescent color and providing excellent transparency (clearness) can be obtained by blending a particle of a specific material selected from hydroxyapatite, calcium carbonate, a metal oxide (except iron oxide) and silica and a specific oil-soluble dye in combination. Based on the finding, the present invention was accomplished.

More specifically, the present invention provides a makeup cosmetic containing (A) at least one oil-soluble dye selected from the group consisting of Red No. 218, Red No. 223 and Orange No. 201, (B) a particle of a specific material selected from hydroxyapatite, calcium carbonate, a metal oxide (except iron oxide) and silica, and (C) an oily component, wherein the makeup cosmetic may further comprise a pigment in an amount of 0.5 mass% or less.

The present invention further provides a cosmetic coloring material to be blended in the makeup cosmetic. The cosmetic coloring material of the present invention contains the components (A), (B) and (C).

### Advantageous Effects of Invention

The makeup cosmetic according to the present invention can produce a bright fluorescent color based on a fluorescent dye intrinsically not producing a fluorescent color by blending a specific oil-soluble dye(s) and a particle of a specific material in combination. The makeup cosmetic according to the present invention can not only provide excellent transparency (clearness) but also produce a bright fluorescent color by suppressing the content of a pigment to a predetermined value or less.

### Description of Embodiments

The present invention will be more specifically described below.

### (1) Makeup cosmetic

The makeup cosmetic of the present invention contains (A) an oil-soluble dye, (B) a particle of a specific material selected from hydroxyapatite, calcium carbonate, a metal oxide (except iron oxide) and silica, and (C) an oily component, as essential components.

### (A) Oil-soluble dye

The oil-soluble dye (component A) to be blended in the makeup cosmetic of the present invention is selected from oil-soluble dyes of the coloring materials called organic synthetic coloring agents in the field of cosmetic, especially an oil-soluble dye of a fluorescein derivative, in particular, at least one or two or more selected from Red No. 218 (tetrachlorotetrabromofluorescein), Red No. 223 (tetrabromofluorescein) and Orange No. 201 (dibromofluorescein).

The content of the oil-soluble dye (component A) in the makeup cosmetic is preferably 0.0001 to 5.0 mass%, and more preferably 0.001% to 3.0 mass% relative to the whole cosmetic.

### (B) Particle of specific material

Component B to be blended in the makeup cosmetic of the present invention is a particle of a specific material selected from hydroxyapatite, calcium carbonate, a metal oxide (except iron oxide) and silica.

The particle of a specific material (component B) to be used in the makeup cosmetic of the present invention is preferably a solid particle, in particular, a powder particle. The shape and size of the particle is not particularly limited and can be selected from those usually blended in cosmetics. Examples thereof include a plate shape (including flakey, thin plate-like, scaly, leaflet-like, mica-like and foil-like), a spherical shape, a columnar shape and massive form.

### (b-1) Hydroxyapatite

Hydroxyapatite (sometimes simply referred to as "HAP") is a calcium phosphate compound having an apatite structure represented by the following formula:

[Chemical Formula 1] **Ca₁₀(PO₄)₆(OH)₂ or Ca_{10-Z}(HPO₄)_{Z}(PO₄)_{6-Z}(OH)₂▪nH₂O**

and a component used as e.g., a polishing/scrubbing agent or a bulking agent in conventional cosmetics.

As the plate-like hydroxyapatite powder suitable for the present invention, a plate-like hydroxyapatite powder having an average particle size (determined by a laser diffraction method) of 1 to 100 µm and a ratio of long-side length to thickness (aspect ratio) of 2 to 200, can be mentioned. As a commercially available product of such plate-like hydroxyapatite powder, for example, "plate-like HAP-SC" (average particle size = 20 to 30 µm; specific surface area = 40 to 80 m²/g; aspect ratio = 2 to 40; manufactured by Taihei Chemical Industrial Co., Ltd.) is known.

As the massive hydroxyapatite powder suitable for the present invention, for example, "KC-apatite" (manufactured by Sumitomo Chemical Co., Ltd.) can be mentioned.

### (b-2) Calcium carbonate

Calcium carbonate is a calcium salt of carbonic acid (CaCO₃) and a component used as e.g., a polishing/scrubbing agent, a buffer, a filler and an opaquer in conventional cosmetics.

Examples of the calcium carbonate powder include a heavy calcium carbonate powder, which is obtained by mechanically crushing limestone and light calcium carbonate (precipitated calcium carbonate) produced by a chemical method. Both of them can be used in the present invention; however, a spherical powder formed of light calcium carbonate having an average particle size of preferably about 1 to 30 µm, more preferably 1 to 20 µm and further preferably 1 to 10 µm is used.

Examples of a commercially available product of the spherical powder of calcium carbonate suitable for the present invention include "Calmaru SCS-M5" (average particle size: 5 µm) and "Calmaru SCS-M2" (average particle size: 2 µm) (both are manufactured by Sakai Chemical Industry Co., Ltd.).

### (b-3) Metal oxide

The metal oxide to be used as component B of the present invention is a component such as titanium oxide, zinc oxide, aluminum oxide (alumina) or zirconium oxide, which is conventionally used as a white pigment or UV scattering agent. A color pigment, iron oxide, is not included.

Titanium oxide may be rutile type or anatase type. Zinc oxide produced by a dry process or a wet process is included.

In the present invention, a metal oxide (except iron oxide) particle preferably has an average particle size of 5 to 200 nm and more preferably 5 to 100 nm.

### (b-4) Silica

As silica (anhydrous silicic acid) to be used as component B of the present invention, a porous silica particle is preferably used.

In the present invention, the average particle size of the silica particle is preferably 0.05 to 10 µm and more preferably 0.05 to 5 µm.

As the porous silica particle to be used in the present invention, for example, a commercially available product such as Satinier M-5 (manufactured by JGC C&C) can be used.

In the present invention, as the particle of a specific material (component B), a particle formed of only a single material selected from hydroxyapatite, calcium carbonate, metal oxide (except iron oxide) and silica, or a particle of an extender pigment such as talc, mica or sericite of which surface is coated with the aforementioned specific material may be used. As the particle of the specific material, a particle of a single material may be blended or a particle of two or more of the specific materials may be blended in combination.

In the makeup cosmetic of the present invention, the content of the particle of a specific material (component B) relative to the whole cosmetic is preferably 0.001 to 30 mass%, more preferably 0.01 to 10 mass%, further preferably 0.01 to 3 mass% and most preferably 0.01 to 1 mass%.

### (C) Oily component

The oily component (component C) to be blended in the makeup cosmetic of the present invention can be selected from oily ingredients usually blended in makeup cosmetics. As the oily component which can be blended, the following examples may be mentioned; however, the oily component is not limited to these.

As a solid oily component, solid fats, waxes, hydrocarbons and higher alcohols may be mentioned. Specific examples thereof include solid fats such as Japanese wax, cocoa butter and hardened castor oil; waxes such as carnauba wax, beeswax, candelilla wax and jojoba wax; hydrocarbon waxes such as polyethylene wax, paraffin wax, ceresin and microcrystalline wax; higher alcohols such as behenyl alcohol, cetanol and batyl alcohol; and silicone wax.

Examples of a liquid oily component include liquid oils such as olive oil, avocado oil, camellia oil, macadamia nut oil, evening primrose oil, jojoba oil, rapeseed oil, egg yolk oil, sesame oil, castor oil, safflower oil, cottonseed oil, soybean oil, tea seed oil, rice bran oil and germ oil; hydrocarbon oils such as squalane and liquid paraffin; ester oils such as isocetyl isostearate, cetyl 2-ethylhexanoate, 2-heptylundecyl palmitate, diisobutyl adipate, 2-hexyldecyl sebacate, isopropyl myristate, 2-octyldodecyl oleate, diisostearyl malate, ethylene glycol di-2-ethylhexanoate, glyceride tri-2-heptylundecanoate, glyceryl diisostearate, glyceryl triisostearate, glyceryl trioctanoate, glyceryl triisopalmitate, trimethylolpropane tri-2-ethylhexanoate and pentaerythritol tetra-2-ethylhexanoate; linear silicone oils such as dimethyl polysiloxane and methylphenyl polysiloxane (diphenylsiloxy phenyl trimethicone); cyclic silicone oils such as octamethylcyclotetrasiloxane and decamethylcyclopentasiloxane; triglycerine; and fluorine-modified oils.

In the makeup cosmetic of the present invention, an ester oil is preferably contained as the oily component (component C). Of them, an ester oil having a refractive index of 1.45 or more and a viscosity of 1,000 cps or more, such as distearyl malate (refractive index: 1.46; viscosity 5,500), polyglyceryl-2 isostearate (refractive index: 1.47; viscosity 6,540) or (isostearic acid/sebacic acid) ditrimethylolpropane oligoester (refractive index: 1.47; viscosity 2,380), is preferably blended.

In the makeup cosmetic of the present invention, in addition to the above essential components (A) to (C), other components (hereinafter referred to also as "optional components"), which are usually blended in makeup cosmetics, can be blended as long as they do not negate the effect of the invention.

Examples of the optional components include components constituting a coloring material and an oil base of a makeup cosmetic and other (optional) components.

Examples of the component constituting a coloring material include an organic synthetic coloring agent (tar color) selected from the group consisting of organic dyes (excluding the oil-soluble dye serving as component A) and organic pigments (including pigment dyestuff and lake); natural colorants; and inorganic pigments (color pigments such as red iron oxide, yellow iron oxide and ultramarine blue, excluding white pigments (metal oxide) corresponding to component B).

Note that, in the makeup cosmetic of the present invention, in order to produce excellent transparency (clearness), the content of a pigment (organic pigment and inorganic pigment) relative to the whole cosmetic is preferably 0.5 mass% or less and further preferably 0.4 mass% or less.

As the component constituting the oil base, an oil thickener or a gelling agent may be mentioned.

Examples of the oil thickener (gelling agent) may include dextrin fatty acid esters, glycerin fatty acid ester and organic modified clay minerals. Examples of the dextrin fatty acid ester include dextrin myristate, dextrin palmitate and dextrin (palmitate/2-ethylhexanoate). Examples of the glycerin fatty acid ester include glyceryl behenate, glyceryl octastearate and glyceryl eicosanoate.

Examples of other (optional) components include a UV absorber, a surfactant, an antioxidant, a fragrance, a preservative, a polyhydric alcohol, a lower alcohol, an inorganic powder (including an extender pigment, a pearlescent pigment, and functional pigments such as a photochromic pigment; except a silica particle corresponding to component B), an organic powder and various active components.

Note that, in the present invention, the extender pigment of which surface is coated with a specific material selected from hydroxyapatite, calcium carbonate, a metal oxide (except iron oxide) and silica serving as component B belongs to component B and is excluded from the extender pigment serving as the optional component.

The content(s) of these optional components can be appropriately controlled depending upon e.g., form and function of the makeup cosmetic.

The makeup cosmetic of the present invention may contain a small amount of water; however, the content of water is preferably as low as possible in view of time-dependent color stability. Accordingly, the content of water in the makeup cosmetic of the present invention is less than 1.5 mass% and preferably 1.0 mass% or less.

The makeup cosmetic of the present invention exerts a special effect that never exerted before, i.e., not only producing a highly bright fluorescent color but also providing excellent transparency (clearness). Because of this, the makeup cosmetic of the present invention is particularly suitable for point-makeup cosmetics including lip cosmetics such as a lipstick and gross, blusher and eye shadow; and especially for an oily makeup cosmetic. Note that, lip creams and makeup bases and foundations which are not primarily intended to produce a color are not included in the makeup cosmetics of the present invention.

The makeup cosmetic of the present invention can be produced by a conventional method usually used for makeup cosmetics. More specifically, the makeup cosmetic of the invention can be produced by adding the essential components (A) to (C) and an optional component(s), mixing/stirring by e.g., a homo mixer while heating, and pouring the mixture into a mold followed by cooling. Preferably, a cosmetic coloring material is produced in advance by stirring/mixing the essential components (A) to (C) while heating, and then subjected to the conventional process together with other components to produce a makeup cosmetic.

### (2) Cosmetic coloring material

The present invention also provides a cosmetic coloring material particularly suitable for blending in the aforementioned makeup cosmetics. The cosmetic coloring material of the present invention contains an oil-soluble dye ("component A"), a particle of at least one specific material selected from hydroxyapatite, calcium carbonate, a metal oxide (except iron oxide) and silica ("component B") and an oily component ("component C").

In the cosmetic coloring material of the present invention, the content of the oil-soluble dye (component A) relative to the whole coloring material is preferably 0.0002 to 10 mass% and more preferably 0.0005 to 8 mass%.

In the cosmetic coloring material of the present invention, the content of the particle (component B) of at least one specific material selected from hydroxyapatite, calcium carbonate, a metal oxide (except iron oxide) and silica relative to the whole coloring material is preferably 0.002 to 60 mass%, more preferably 0.005 to 50 mass%, further preferably 0.01 to 6 mass% and most preferably 0.02 to 3 mass%.

The oily component serving as a constituent of the cosmetic coloring material of the present invention may constitute a part or whole of the oily component (component C) to be blended in the makeup cosmetic of the present invention and is preferably selected from the aforementioned oils that can dissolve or disperse the oil-soluble dye (component A).

Examples of the oily component suitably used for the coloring material of the present invention may include, but are not limited to, paraffin, squalane, isoparaffin, dimethicone, methylphenylpolysiloxane, di(isostearyl/phytosteryl) dilinoleate dimer, phytosteryl/behenyl dilinoleate dimer, phytosteryl/octyldodecyl lauroyl glutamate, glyceryl tri2-ethylhexanoate, glyceryl triisostearate, glyceryl tri(hydrogenated rosin/isostearate), diisostearyl malate, pentaerythrityl tetraethylhexanoate, dipentaerythrityl hexahydroxystearate and polyglyceryl-2 triisostearate.

In the cosmetic coloring material of the present invention, the content of the oily component is preferably 30 to 99.9978 mass%, more preferably 42 to 99.9945 mass% and further preferably 80 to 99.50 mass%.

If necessary, e.g., an oil thickener (gelling agent) as mentioned above may be contained.

The cosmetic coloring material of the present invention is preferably contained at 10 to 60 mass%, and preferably 20 to 50 mass% in the makeup cosmetic producing a fluorescent color to which the cosmetic coloring material is added.

The coloring material of the present invention can be produced by, for example, dissolving or dispersing an oil-soluble dye (component A) in an oily component (component C), adding a particle of a specific material (component B) to the mixture and stirring/mixing the mixture while heating. The heating temperature preferably falls within the range of 80°C to 140°C, more preferably 90°C to 130°C and further preferably 100°C to 120°C. In the coloring material and makeup cosmetic containing the coloring material of the present invention, an oil-soluble dye (A) and a particle of a specific material (B) are present in an oily component in the state of being directly dissolved or dispersed, and can be clearly distinguished from a cosmetic containing, e.g., an oil-soluble dye carried on a solid carrier (e.g., solid particle).

### Examples

Now, the present invention will be more specifically described by way of Examples; however, the present invention is not limited by these. Note that, unless otherwise specified, the content is expressed by mass% relative to the whole amount.

### (Examples and Comparative Examples)

Lipstick cosmetics having the compositions listed in the following Tables 1 and 2 were prepared.

In each example, lipstick cosmetics were prepared in accordance with the following two processes.

(Process 1) The components to be blended (listed in Tables) were combined, heated to 80 to 140°C, stirred/mixed, defoamed, poured in lipstick containers and cooled to 5°C.

(Process 2) Of the components listed in Tables, oily components (paraffin, microcrystalline wax and methylphenyl silicone in Table 1; polyethylene, microcrystalline wax, diphenylsiloxy phenyl trimethicone in Table 2), an oil-soluble dye and a particle of hydroxyapatite, calcium carbonate, a metal oxide (except iron oxide) or silica or nylon powder were used in their whole amounts. The dye was dissolved or dispersed in the oily component and particles of hydroxyapatite, calcium carbonate, a metal oxide (except iron oxide) or silica or a nylon powder were added and stirred/mixed while heating to 100 to 120°C to prepare a cosmetic coloring material. Thereafter, the resultant cosmetic coloring material and the remaining components were mixed/stirred and defoamed while heating to 80 to 140°C, and cooled to 5°C.

The lipstick cosmetics and coloring materials of individual examples were evaluated by 10 experts (panelists) in accordance with the following criteria.

### (1) Fluorescent color

Remarkable fluorescent color is observed: 4
Fluorescent color is observed: 3
Color is produced but not a fluorescent color: 2
Color is not observed: 1

### (2) Transparency (clearness)

Remarkably transparent (clear): 4
Transparent (clear): 3
Slightly transparent (clear): 2
Not transparent (clear) (covering power is strong): 1

Evaluation results according to the above criteria were rated as follows and the rating results of individual examples are shown together in Table 1.
A: Total score is 35 to 40
   (as to the fluorescent color, a case obtaining a total score of 35 to 37 was rated as A⁻; and a case obtaining a total score of 38 to 40 was rated as A⁺)
B: Total score is 25 to 34
C: Total score is 15 to 24
D: Total score is 10 to 14

### (3) Color stability with time

Cosmetics prepared from the compositions shown in Table 1 were stored at room temperature and change in color was evaluated based on visual observation.
A: Color change was not observed at all.
B: Color slightly changed but the change was not a problem in a practical point of view.
C: Color remarkably changed.

**[Table 1]**

| | Component name | | Example | | | | | Comparative Example | | |
|---|---|---|---|---|---|---|---|---|---|---|
| | | | 1 | 2 | 3 | 4 | 5 | 1 | 2 | 3 |
| Solid oily component | Paraffin | | 4 | 4 | 4 | 4 | 4 | 4 | 4 | 4 |
| | Microcrystalline wax | | 2 | 2 | 2 | 2 | 2 | 2 | 2 | 2 |
| | Candelilla wax | | 1 | 1 | 1 | 1 | 1 | 1 | 1 | 1 |
| Liquid oily component | Distearyl malate | | 20 | 20 | 20 | 20 | 20 | 20 | 20 | 20 |
| | Diphenylsiloxy phenyl trimethicone | | 41.9 | 41.9 | 41.9 | 41.8 | 40.9 | 41.9 | 41.1 | 40.4 |
| | Hydrogenated polyisobutene | | 10 | 10 | 10 | 10 | 10 | 10 | 10 | 10 |
| | Di (isostearyl/phytosteryl) dilinoleate dimer | | 10 | 10 | 10 | 10 | 10 | 10 | 10 | 10 |
| | Polyglyceryl-2 triisostearate | | 10 | 10 | 10 | 10 | 10 | 10 | 10 | 10 |
| Dye | Red No. 218 | | 0.1 | 0.1 | 0.1 | 0.1 | 0.1 | 0.1 | 0.1 | 0.1 |
| Pigment | Red No. 202 | | - | - | - | 0.1 | - | - | 0.8 | - |
| Component B | Plate-like hydroxyapatite⁽¹⁾ | | 1 | - | - | 1 | 1 | - | 1 | 1 |
| | Massive hydroxyapatite⁽²⁾ | | - | 1 | - | - | - | - | - | - |
| | Spherical calcium carbonate⁽³⁾ | | - | - | 1 | - | - | - | - | - |
| | Nylon | | - | - | - | - | - | 1 | - | - |
| Water | Ion-exchange water | | | | | | 1.0 | | | 1.5 |
| Total | | | 100 | 100 | 100 | 100 | 100 | 100 | 100 | 100 |
| Fluorescent color | | Process 1 | A⁻ | A⁻ | A⁻ | A⁻ | A⁻ | B | c | A⁻ |
| | | Process 2 | A⁺ | A⁺ | A⁺ | A⁺ | A⁺ | B | c | A⁺ |
| Transparency (clearness) | | | A | A | A | A | A | C | C | A |
| Color stability with time | | | A | A | A | A | B | A | A | C |

| | | | | | | | | | | |
|---|---|---|---|---|---|---|---|---|---|---|
| (1) "Plate-like HAP-SC" (manufactured by Taihei Chemical Industrial Co., Ltd.) (2) "KC-apatite" (manufactured by SUMITOMO CHEMICAL Co., Ltd.) (3) "Calmaru SCS-M5"(manufactured by Sakai Chemical Industry Co., Ltd.) | | | | | | | | | | |

The cosmetic coloring materials of Examples 1 to 4 containing hydroxyapatite or calcium carbonate and prepared in accordance with process 2 produced bright fluorescent colors and had clearness (evaluation of fluorescent color: A⁺); however, the coloring material of Comparative Example 1 containing nylon powder (nylon SP-500, manufactured by Toray Industries Inc., molecular weight: 10,000 to 100,000, particle size: 10 µm or less, spherical powder) described in Patent Document 3 produced pink fluorescent color but poor, and hiding power was too strong to obtain transparency (clearness) (evaluation of fluorescent color: B).

As is apparent from the results shown in Table 1, in the makeup cosmetics of Examples 1 to 4 containing a specific oil-soluble dye (component A) and particles of a specific material (component B), a bright fluorescent color was observed regardless of the process and transparency (clearness) of the cosmetics was excellent. Note that, the fluorescent colors in the cases where makeup cosmetics were produced in accordance with Process 2 were brighter than Process 1. Furthermore, even if a small amount of water (1 mass%) was added, color stability was satisfactory in a practical point of view (Example 5).

In contrast, in the makeup cosmetic of Comparative Example 1 containing nylon powder in place of the particles of a specific material and produced in accordance with either of Process 1 and Process 2, the fluorescent color produced was poor and no transparency (clearness) was obtained. In the makeup cosmetic of Comparative Example 2 containing more than 0.5 mass% of an organic pigment (Red No. 202), neither transparency (clearness) nor fluorescent color was obtained. In the makeup cosmetic of Comparative Example 3 containing 1.5 mass% of water, color significantly changed.

**[Table 2]**

| | Component name | | | | | | | | |
|---|---|---|---|---|---|---|---|---|---|
| | | 6 | 7 | 8 | 9 | 10 | 11 | 12 | 13 |
| Solid oily component | Polyethylene | 12 | 12 | 12 | 12 | 12 | 12 | 12 | 12 |
| | Microcrystalline wax | 2 | 2 | 2 | 2 | 2 | 2 | 2 | 2 |
| | Candelilla wax | 1 | 1 | 1 | 1 | 1 | 1 | 1 | 1 |
| Liquid oily component | Distearyl malate | 10 | 10 | 10 | 10 | 10 | 10 | 10 | 10 |
| | Diphenylsiloxy phenyl trimethicone | 41.8 5 | 41.57 8 | 42.0 5 | 39. 5 | 40.5 9 | 43.3 5 | 43.3 4 | 43.6 5 |
| | Triethylhexanoin | 10 | 10 | 10 | 10 | 10 | 10 | 10 | 10 |
| | Hydro genated polyisobutene | 10 | 10 | 10 | 10 | 10 | 10 | 10 | 10 |
| | Dipentaerythrityl hexahydroxystearat e | 10 | 10 | 10 | 10 | 10 | 10 | 10 | 10 |
| Dye | Red No. 218 | 0.05 | 0.3 | 0.1 | - | 0.05 | - | - | 0.05 |
| | Red No.223 | - | 0.01 | 0.05 | 0.2 | - | 0.05 | 0.01 | - |
| | Orange No. 201 | - | 0.002 | - | 2 | 1 | - | 0.05 | - |
| Pigment | iron oxide | - | - | - | - | 0.01 | - | - | - |
| | Red No. 202 | - | - | - | - | 0.2 | - | - | - |
| Component B | Hydroxyapatite⁽¹⁾ | 1 | 2 | 1 | - | 1 | - | - | - |
| | Calcium carbonate⁽²⁾ | - | - | - | 1 | 0.05 | - | - | - |
| | Zinc oxide⁽³⁾ | - | - | - | - | - | 1 | - | - |
| | Titanium oxide⁽⁴⁾ | - | - | - | - | - | - | 0.5 | - |
| | Silica ⁽⁵⁾ | 0.5 | - | 0.5 | - | - | - | - | 1 |
| Powder | Calcium stearate | 0.5 | 0.01 | 1 | 0.2 | 0.5 | - | - | - |
| | Aluminum stearate | - | - | 0.2 | 1 | - | 0.5 | - | - |
| | Magnesium stearate | - | - | - | - | 0.5 | - | - | 0.2 |
| | Titanium mica | 1 | - | - | 1 | - | - | - | - |
| | Sericite | - | 1 | - | - | 1 | - | 1 | - |
| | Fragrance | 0.05 | 0.05 | 0.05 | 0.0 5 | 0.05 | 0.05 | 0.05 | 0.05 |
| | Tocopherol | 0.05 | 0.05 | 0.05 | 0.0 5 | 0.05 | 0.05 | 0.05 | 0.05 |
| Total | | 100 | 100 | 100 | 100 | 100 | 100 | 100 | 100 |
| Fluorescent color | | A | A | A | A | A | A | A | A |
| Transparency (clearness) | | A | A | A | A | A | A | A | A |

| | | | | | | | | | |
|---|---|---|---|---|---|---|---|---|---|
| (1) "Plate-like HAP-SC" (manufactured by Taihei Chemical Industrial Co., Ltd.) (2) "Calmaru SCS-M5" (manufactured by Sakai Chemical Industry Co., Ltd.) (3) Zinc oxide, AZO-BS (manufactured by SEIDO CHEMICAL INDUSTRY CO., LTD.), particle size: 0.02 to 0.06 µm (4) Titanium oxide MT-014V (manufactured by TAYCA), particle size: 0.01 to 0.05 µm (5) Satinier M-5 (manufactured by JGC C&C), particle size: 4 to 7 µm | | | | | | | | | |

Table 2 shows Examples 6 to 13 different in combination and content of (A) oil-soluble dyes and (B) the particles of specific materials. As is apparent from the evaluation results described in Table 2, the cosmetics of Examples 6 to 13 were satisfactory both in fluorescence color and transparency (clearness).

Other formulations of the makeup cosmetics according to the present invention will be described below. In any of the makeup cosmetics, bright fluorescent color and clearness were obtained.

**Formulation 1: Lip gloss**

| Component | Content (mass%) |
|---|---|
| 1. Liquid paraffin | 10.0 |
| 2. Polyisobutene | 10.0 |
| 3. Diisostearyl malate | 10.0 |
| 4. Dextrin palmitate | 10.0 |
| 5. Plate-like hydroxyapatite ^{(a)} | 1.0 |
| 6. Orange No. 201 | 0.2 |
| 7. Iron oxide | 0.1 |
| 8. Red No. 202 | 0.1 |
| 9. Titanium mica | 2.0 |
| 10. Tocopherol | proper amount |
| 11. Fragrance | proper amount |
| 12. Diphenylsiloxy phenyl trimethicone | balance |

| | |
|---|---|
| (a) "Plate-like HAP-SC" (manufactured by Taihei Chemical Industrial Co., Ltd.) | |

### (Production method)

Components 1 to 5 and 8 (each in the whole amount) were combined and stirred/mixed while heating to 100 to 120°C to prepare a cosmetic coloring material. Thereafter, the resultant cosmetic coloring material and the remaining components (6, 7 and 9 to 12) were combined, mixed/stirred and defoamed while heating to 80 to 140°C, and cooled.

**Formulation 2: Eye shadow**

| Component | Content (mass%) |
|---|---|
| 1. Talc | balance |
| 2. Mica | 20.0 |
| 3. Boron nitride | 10.0 |
| 4. Synthetic mica | 10.0 |
| 5. Mica coated with titanium oxide | 5.0 |
| 6. Iron oxide | 2.0 |
| 7. Squalane | 2.0 |
| 8. Diethyl polysiloxane | 2.0 |
| 9. Sorbitan monooleate | 0.5 |
| 10. Red No. 218 | 0.01 |
| 11. Calcium carbonate ^{(b)} | 1.5 |
| 12. Preservative | proper amount |
| 13. Fragrance | proper amount |

| | |
|---|---|
| (b) "Calmaru SCS-M5" (manufactured by Sakai Chemical Industry Co., Ltd.) | |

### (Production method)

Components 1 to 6, 12 and 13 were ground and mixed. To this, a cosmetic coloring material prepared by mixing Components 7 to 11 while heating at 100 to 120°C was added and molded in a pan to obtain an eye shadow.

**Formulation 3: Blusher**

| Component | Content (mass%) |
|---|---|
| 1. Talc | balance |
| 2. Polymethyl methacrylate | 1.0 |
| 3. Silica | 2.0 |
| 4. Boron nitride | 3.0 |
| 5. Mica | 10.0 |
| 6. Isoparaffin | 3.0 |
| 7. Glyceryl tri-2-ethylhexanoate | 1.0 |
| 8. Sorbitan monooleate | 1.0 |
| 9. Iron oxide | 3.0 |
| 10. Red No. 223 | 0.1 |
| 11. Red No. 218 | 0.02 |
| 12. Massive hydroxyapatite ^{(c)} | 3.0 |
| 13. Preservative | proper amount |
| 14. Fragrance | proper amount |

| | |
|---|---|
| (c) "KC-apatite" (manufactured by SUMITOMO CHEMICAL Co., Ltd.) | |

### (Production method)

Components 1 to 5, 9, 13 and 14 were ground and mixed. To this, a cosmetic coloring material prepared by mixing components 6 to 8 and 10 to 12 while heating at 100 to 120°C, was added and molded in a pan to obtain a blusher.

## Claims

1. A makeup cosmetic comprising
(A) at least one or two or more kinds of oil-soluble dye selected from the group consisting of Red No. 218, Red No. 223 and Orange No. 201,
(B) a particle of a specific material selected from hydroxyapatite, calcium carbonate, a metal oxide (except iron oxide) and silica, and
(C) an oily component,
wherein said makeup cosmetic may further comprise a pigment in an amount of 0.5 mass% or less; and wherein said makeup cosmetic may further comprise water in an amount of less than 1.5 mass%.

2. The makeup cosmetic according to Claim 1, wherein said particle of a specific material (B) is selected from plate-like powder of hydroxyapatite, a massive powder of hydroxyapatite, a spherical powder of calcium carbonate, a titanium oxide powder, a zinc oxide powder and a silica powder.

3. The makeup cosmetic according to Claim 1 or 2, wherein a content of said oil-soluble dye (A) relative to a whole cosmetic is 0.0001 to 5 mass% and a content of said particle of a specific material (B) relative to the whole cosmetic is 0.001 to 30 mass%.

4. The makeup cosmetic according to any one of Claims 1 to 3 wherein: said makeup cosmetic is a lip cosmetic.

5. A coloring material for a makeup cosmetic comprising
(A) at least one oil-soluble dye selected from the group consisting of Red No. 218, Red No. 223 and Orange No. 201 at 0.0002 to 10 mass%,
(B) a particle of a specific material selected from hydroxyapatite, calcium carbonate, a metal oxide (except iron oxide) and silica at 0.002 to 60 mass%, and
(C) an oily component at 30 to 99.9978 mass%.

6. A makeup cosmetic containing the coloring material according to Claim 5.
